# EUROPEAN PATENT APPLICATION

(11) **EP 4 541 414 A1**
(43) Date of publication of application: **23.04.2025**
(21) Application number: 22946972.1
(22) Date of filing: 06.09.2022
(51) Int. Cl.: A61N 1/36, A61N 1/04, A61N 5/06, A61M 21/00

(54) **STIMULUS APPLYING DEVICE**

(30) Priority: 15.06.2022 KR 20220072656
(71) Applicant: Neurogrin Inc., Seoul 02738 (KR)
(72) Inventor: KIM, Sun Kwang, Seoul 06990 (KR); CHOI, Seunghwan, Seoul 01804 (KR); YOON, Heera, Gimpo-si, Gyeonggi-do 10071 (KR); CHUNG, Geehoon, Seoul 02451 (KR)
(74) Representative: AWA Sweden AB
(86) International application number: PCT/KR2022/013378
(87) International publication number: WO 2023/243767

(57) **Abstract**

Disclosed is a stimulus applying apparatus. A stimulus applying apparatus according to an embodiment of the present invention comprises: stimulus applying units configured to apply a stimulus to a target portion; and a housing coupled to the stimulus applying unit. The housing comprises: a first housing arranged on one side of the target portion and accommodating a stimulus applying unit; and a second housing arranged on the other side of the target portion and accommodating a stimulus applying unit. The stimulus applying unit accommodated in the first housing and the stimulus applying unit accommodated in the second housing may be configured to apply magnetic attractive force to each other.

## Description

### [Technical Field]

The present invention relates to a stimulus applying apparatus, and more specifically to a stimulus applying apparatus that can provide a stimulus for cognitive function recovery and can be easily attached to a target portion.

### [Background Art]

Vascular dementia, which is the most severe form of vascular cognitive disorders that are cognitive disorders accompanying cerebrovascular disease, occurs when brain tissue is damaged by cerebrovascular disease. Vascular dementia is characterized by impaired memory.

Dementia is a degenerative disease that exhibits clinical symptoms that result in acquired loss of intellectual ability, cognitive impairment and gradual deterioration of behavior and personality, and it is one of the serious social problems along with the rapid aging of the population, and is a serious social problem in the construction of a health and welfare nation. Additionally, it is a representative disease of old age that is emerging, and the resulting increase in medical costs for additional caregivers is holding back the national economy.

To date, medicaments used for Alzheimer's dementia, such as donepezil and galantamine, have been proposed as pharmacological treatments, but their effectiveness is only half that of that seen in Alzheimer's dementia (Battle et al., Cochrane Database of Systematic Reviews 2021). In addition, vagus nerve stimulus technology has been utilized in the form of surgical implantation in the neck for cognitive dysfunction, but it is invasive and difficult to apply in most cases.

Accordingly, the inventors of the present invention have identified a need for a non-invasive and detachable therapeutic technology for recovering cognitive function damaged by vascular dementia.

Korean Patent Application Laid-Open No. 10-2014-0037803 discloses a machine, system and method for treating medical diseases. Specifically, it discloses a machine, system and method for treating medical diseases that can stimulate the skin of the superficial branch of the trigeminal nerve that is located extracranially on the face.

However, the machine, system and method for treating medical diseases disclosed in the related art document are configured to apply a stimulus to a body part using a band-shaped electrode assembly. Therefore, there is a limitation in that it is difficult to apply the band-shaped electrode assembly to body parts where it is difficult to wear.

Korean Registered Patent No. 10-2386264 discloses non-invasive nerve stimulation through a portable device. Specifically, it discloses a non-invasive portable device that can improve a patient's condition by applying electrical stimuli to one or more nerves.

However, the non-invasive nerve stimulation disclosed in the related art document also assumes that it is coupled to a body part through a band or the like. Accordingly, the related art document does not suggest a method for attaching a device for applying stimuli to a small body part, when it is intended to apply stimuli to the small body part.
Korean Publication Application Laid-Open No. 10-2014-0037803 (March 27, 2014)
Korean Registered Patent No. 10-2386264 (April 13, 2022)

### [Disclosure]

### [Technical Problem]

The present invention is intended to solve the above problems, and an object of the present invention is to provide a stimulus applying apparatus that can be easily coupled to an area to which a stimulus is to be applied.

Another object of the present invention is to provide a stimulus applying apparatus that can be easily removed after being attached to the above area.

Still another object of the present invention is to provide a stimulus applying apparatus that can restore cognitive function in a non-invasive manner.

Still another object of the present invention is to provide a stimulus applying apparatus that can be easily worn on the above area.

Still another object of the present invention is to provide a stimulus applying apparatus that is non-invasive, is detachable and can be easily applied, and can be used continuously by reducing the risks associated with transplant surgery and the like, when electroceuticals are used.

However, the technical problem to be achieved by the present invention is not limited to the problems mentioned above, and other problems that are not mentioned will be clearly understood by those skilled in the art from the description below.

### [Technical Solution]

According to an aspect of the present invention, provided is a stimulus applying apparatus, including stimulus applying units which are configured to apply a stimulus to a target portion; and a housing which is coupled to the stimulus applying unit, wherein the housing includes a first housing which is arranged on one side of the target portion and accommodates the stimulus applying unit; and a second housing which is arranged on the other side of the target portion and accommodates the stimulus applying unit, and wherein the stimulus applying unit accommodated in the first housing and the stimulus applying unit accommodated in the second housing are configured to apply magnetic attractive force to each other.

**In** this case, the stimulus applying apparatus may be provided, wherein the stimulus applying unit may include a magnetic member which is accommodated in the housing and includes a stimulating electrode which is configured to apply the stimulus to the target portion, and wherein the magnetic member may be located to be biased on one side of the housing facing the target portion.

**In** addition, the stimulus applying apparatus may be provided, wherein the housing includes an applying member accommodating unit that accommodates the magnetic member and is located adjacent to the target portion, and wherein the applying member accommodating unit includes an accommodating space which is formed to be recessed on one surface facing the target portion (T) to accommodate the magnetic member.

**In** this case, the stimulus applying apparatus may be provided, wherein the housing includes a wire-guiding unit which is continuous with the applying member accommodating unit and is formed to extend in a direction opposite to the applying member accommodating unit, and wherein the wire-guiding unit includes guide hollows that are located therein and are formed to penetrate along an extension direction thereof such that one end in the extension direction communicates with the accommodating space, and the other end in the extension direction communicates with the outside.

In addition, the stimulus applying apparatus may be provided, wherein the stimulus applying unit includes a control unit which is electrically connected to the magnetic member and is configured to apply power and control signals to the magnetic member.

In this case, the stimulus applying apparatus may be provided, wherein the control unit is located outside the housing, and includes a conductive wire member which extends between the control unit and the magnet member and is configured to conduct electricity with the control unit and the magnet member, respectively.

In addition, the stimulus applying apparatus may be provided, wherein the stimulus applying unit includes a battery which is accommodated inside the housing to be adjacent to the magnetic member, and is configured to apply power and control signals to the magnetic member.

In addition, according to another aspect of the present invention, provided is a stimulus applying apparatus, including stimulus applying units which are configured to apply a stimulus to a target portion; and a housing which is coupled to the stimulus applying unit, wherein the housing includes a main housing which accommodates the stimulus applying unit, covers one side of the target portion, and is inserted into an opening that is formed in the target portion; a sub-housing which accommodates the stimulus applying unit, and is located to be adjacent to the other side of the target portion; and a holding member which extends between the main housing and the sub-housing, and is configured to be mounted on another side of the target portion.

In this case, the stimulus applying apparatus may be provided, wherein the main housing includes an insertion protrusion unit which is formed to protrude toward the target portion, and is inserted into the opening of the target portion, and wherein the stimulus applying unit is arranged on the insertion protrusion unit, and is configured to apply the stimulus to a part that is adjacent to the opening of the target portion.

In addition, the stimulus applying apparatus may be provided, wherein the stimulus applying unit includes a magnetic member which includes a stimulating electrode and is configured to apply an electrical stimulus to the target portion; and a light stimulus applying member which is configured to apply an optical stimulus to the target portion.

In this case, the stimulus applying apparatus may be provided, wherein the magnetic member is arranged in any one or more of the main housing and the sub-housing, and wherein the light stimulus applying member is arranged to be adjacent to the magnet member in any one or more of the main housing and the sub-housing.

In addition, the stimulus applying apparatus may be provided, wherein the magnetic member is arranged in any one or more of the main housing and the sub-housing, and wherein the light stimulus applying member is arranged to be adjacent to the magnet member in any one or more of the main housing and the sub-housing.

In this case, the stimulus applying apparatus may be provided, wherein the stimulus applying unit includes a magnetic member which includes a stimulating electrode and is configured to apply an electrical stimulus to the target portion; and a control unit which is electrically connected to the magnetic member and is configured to apply power and control signals to the magnetic member, wherein the control unit is arranged outside the housing and is configured to conduct electricity to the magnet member by a conductive wire member.

In addition, the stimulus applying apparatus may be provided, wherein the stimulus applying unit includes a magnet member which is accommodated in the housing, includes a stimulating electrode and is configured to apply an electrical stimulus to the target portion; and a battery which is accommodated in the housing to be adjacent to the magnetic member, is electrically connected to the magnetic member and is configured to apply power and control signals to the magnetic member.

### [Advantageous Effects]

According to the above configuration, the stimulus applying apparatus according to an exemplary embodiment of the present invention can be easily coupled to an area to which a stimulus is to be applied.

In addition, according to the above configuration, the stimulus applying apparatus according to an exemplary embodiment of the present invention can be easily removed after being attached to the above area.

In addition, according to the above configuration, the stimulus applying apparatus according to an exemplary embodiment of the present invention can restore cognitive function in a non-invasive manner.

In addition, according to the above configuration, the stimulus applying apparatus according to an exemplary embodiment of the present invention can be easily worn on the above area.

In addition, according to the above configuration, the stimulus applying apparatus according to the embodiment of the present invention is non-invasive and detachable, and thus, it can be easily applied, and there is little risk involved in transplant surgery and the like, thereby facilitating the recovery of cognitive function damaged due to vascular dementia. Accordingly, the present invention can be used as a continuous therapeutic technology for recovering the cognitive function of patients.

The effects of the present invention are not limited to the effects described above, and it should be understood to include all effects that can be inferred from the configuration of the invention described in the Detailed Description of the Invention or claims.

### [Description of Drawings]

FIG. 1 is a usage status diagram illustrating a stimulus applying apparatus according to an exemplary embodiment of the present invention.
FIG. 2 is an exploded perspective view illustrating the stimulus applying apparatus of FIG. 1.
FIG. 3 is a rear view (a) and a top view (b) illustrating the stimulus applying apparatus of FIG. 1.
FIG. 4 is a block diagram illustrating the configuration of the stimulus applying apparatus of FIG. 1.
FIG. 5 is a usage status diagram illustrating a stimulus applying apparatus according to another exemplary embodiment of the present invention.
FIG. 6 is a left side view (a) and right side view (b) illustrating the stimulus applying apparatus of FIG. 5.
FIG. 7 is a block diagram illustrating the configuration of the stimulus applying apparatus of FIG. 5.
FIG. 8 is a schematic diagram of a surgical method for causing vascular dementia.

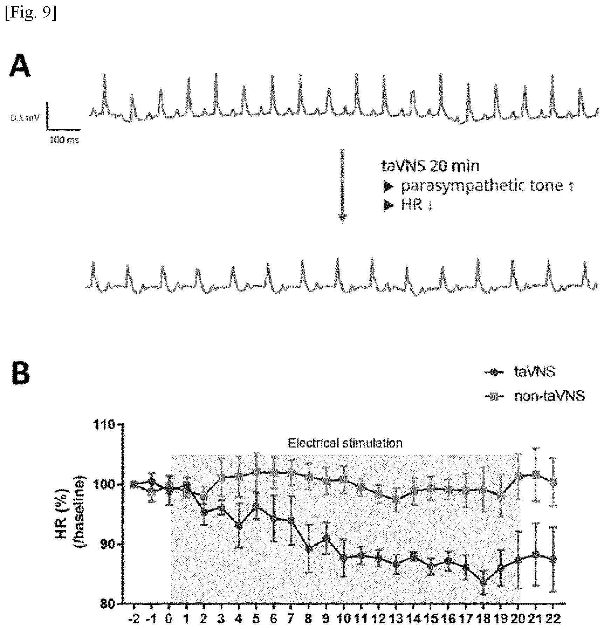

FIG. 9 shows the results of confirming the heart rate reduction caused by transcutaneous vagus nerve stimuli.
FIG. 10 shows the results of memory impairment in vascular dementia induced by temporary common carotid artery ligation.
FIGS. 11a to 11f show the results of confirming cognitive function recovery by transcutaneous vagal nerve stimuli (taVNS) in vascular dementia animals showing memory impairment.
FIG. 12 shows the results of confirming the activation of glymphatic circulation by transcutaneous vagus nerve stimuli.

### [Modes of the Invention]

Hereinafter, with reference to the attached drawings, the exemplary embodiments of the present invention will be described in detail so that those skilled in the art can easily practice the present invention. The present invention may be implemented in many different forms and is not limited to the exemplary embodiments described herein. In order to clearly describe the present invention, parts that are not relevant to the description have been omitted in the drawings, and the same or similar components are assigned the same reference numerals throughout the specification.

The words and terms used in the present specification and claims are not to be construed as limited in their usual or dictionary meanings, but according to the principle that the inventor can define terms and concepts in order to explain his or her invention in the best way, it must be interpreted with meaning and concepts that are consistent with technical ideas.

Therefore, the exemplary embodiments described in the present specification and the configuration shown in the drawings correspond to a preferred exemplary embodiment of the present invention, and do not represent the entire technical idea of the present invention, and thus, there may be various equivalents and modifications to the corresponding configuration at the time of filing of the present invention.

In the following description, in order to clarify the characteristics of the present invention, the descriptions of some components may be omitted.

The term "electrical conduction" used in the following description means that one or more members are connected to each other to transmit current or electrical signals. In an exemplary embodiment, electrical conduction may be formed in a wired form using a conductor member, or in a wireless form such as Bluetooth, Wi-Fi or RFID.

As used herein, the term 'electroceutical' is a compound word of electronics and pharmaceutical and refers to an electronic device that treats diseases by controlling signals generated in the brain and nerve cells. As used herein, the term 'electroceutical' may be a medical device aimed at providing a drug-like therapeutic effect on various diseases, including incurable diseases, by utilizing physical stimuli with minimal side effects in order to influence and modify the body's biological functions or pathological processes.

In particular, the electroceutical may be provided as stimulus applying apparatuses 10, 20 according to each exemplary embodiment of the present invention, which will be described below.

Electroceuticals can minimize side effects by selecting and stimulating only the specific part that needs treatment, and customized treatment is possible because the stimulus conditions can be individualized by considering the characteristics of a subject. In case of emergency, it has an advantage that the efficacy of the medicine can be stopped immediately. The stimulating area may be a local area of the body, a specific cell or the central or peripheral nervous system, and preferably, cognitive function can be restored by stimulating the specific areas of the brain listed above.

The term "target portion (T)" used in the following description refers to any area that receives a stimulus applied by the stimulus applying apparatuses 10, 20 according to an exemplary embodiment of the present invention. In an exemplary embodiment, the target region (T) may be defined as a part of the human or animal body. In an exemplary embodiment, the target portion (T) may be an ear area.

The term "stimulus" used in the following description refers to any type of signal applied to the target portion (T) to achieve the desired effect. In an exemplary embodiment, the stimulus may be electrical, such as by the electrical conduction of an electric current. In other exemplary embodiments, the stimulus may be in an optical form through the irradiation of light, or in an acoustic form through sound and the like.

Hereinafter, the stimulus applying apparatuses 10, 20 according to exemplary embodiments of the present invention will be described by assuming that they are worn on a specific body part of the wearer, for example, the ear area. Alternatively, in order to maximize the effect of the stimulus applying apparatuses 10, 20 according to exemplary embodiments of the present invention, the stimulus applying apparatuses 10, 20 according to exemplary embodiments of the present invention may be operated in conjunction with other devices.

For example, the stimulus applying apparatuses 10, 20 according to exemplary embodiments of the present invention may be operated in conjunction with brain/biological signal recording devices, such as Electroencephalography (EEG), Magnetic Encephalography (MEG), Near-Infrared Spectroscopy (NIRS), Photoplethysmography (PPG), Galvanic Skin Response (GSR) and the like.

Furthermore, the stimulus applying apparatuses 10, 20 according to exemplary embodiments of the present invention may be operated in conjunction with various wearable devices (VR, headphones, earphones, glasses, bracelets for non-invasive transcutaneous stimulus, accessories such as anklets, smart watches, etc.).

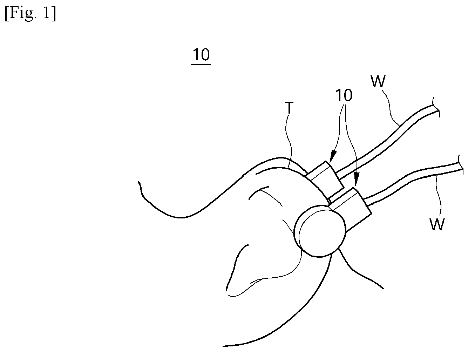

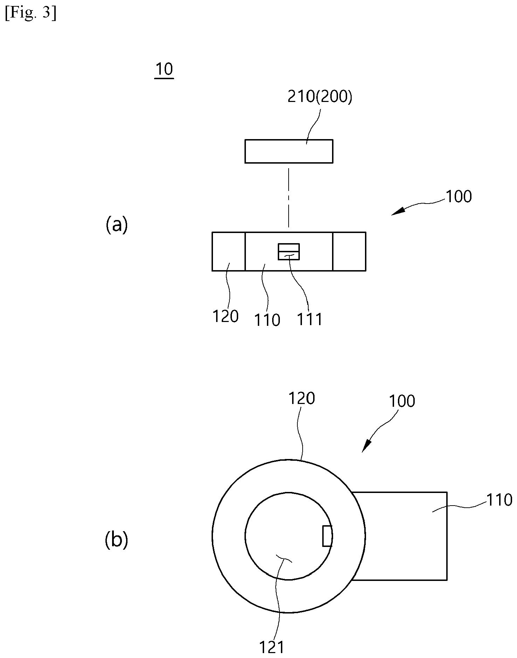

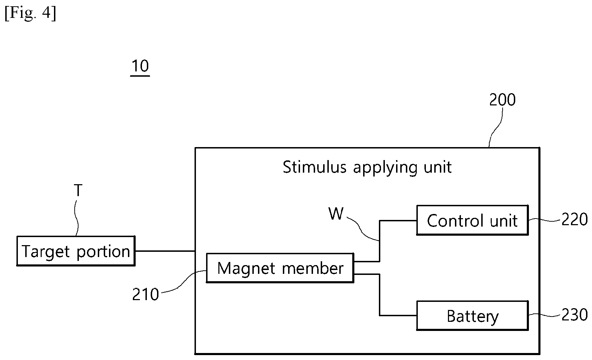

Referring to FIGS. 1 to 4, the stimulus applying apparatus 10 according to an exemplary embodiment of the present invention is illustrated. The stimulus applying apparatus 10 according to the illustrated exemplary embodiment may be detachably coupled to a target portion (T) and configured to apply a stimulus to the target portion (T).

**In** the illustrated exemplary embodiment, the stimulus applying apparatus 10 includes a housing 100 and a stimulus applying unit 200.

The housing 100 forms the external shape of the stimulus applying apparatus 10. The housing 100 is a part where the stimulus applying apparatus 10 is coupled to the target portion (T). The housing 100 may be formed to have a smaller size compared to the target portion (T).

The housing 100 may be made of a lightweight and highly rigid material. This is to prevent a situation where the stimulus applying apparatus 10 coupled to the target portion (T) is arbitrarily separated due to its self-weight.

The housing 100 may be formed of a non-conductive material. This is to prevent a situation where the stimulus to be delivered to the target portion (T) arbitrarily leaks into the housing 100.

In an exemplary embodiment, the housing 100 may be formed of a synthetic resin material such as insulating plastic and the like.

There may be a plurality of housings 100. The plurality of housings 100 are respectively coupled to the target portion (T), and may be arranged in different positions. In an exemplary embodiment, the plurality of housings 100 may be arranged to face each other with the target portion (T) interposed therebetween.

In the illustrated exemplary embodiment, two housings 100 are provided, including a first housing 100a and a second housing 100b. The first housing 100a and the second housing 100b are arranged to face each other with the target portion (T) interposed therebetween. In this case, the first housing 100a and the second housing 100b are respectively coupled to the target portion (T) by a magnetic force applied by the magnet member 210 of the stimulus applying unit 200, which will be described below.

The first housing 100a and the second housing 100b differ in directions in which they are coupled to the target portion (T), but their structures and functions are the same. Accordingly, in the following description of the same technical features, it will be collectively referred to as a "housing 100."

The housing 100 is coupled to a conductive wire member (W). The magnetic pole applying unit 200 accommodated inside the housing 100 may be electrically connected to the outside through the conductive wire member (W).

The housing 100 is coupled to the stimulus applying unit 200. The stimulus applying unit 200 may be configured to apply a stimulus to the target portion (T) while being accommodated in the housing 100.

In the illustrated exemplary embodiment, the housing 100 includes a wire-guiding unit 110 and an applying member accommodating unit 120.

The wire-guiding unit 110 is a part where the housing 100 is coupled to the conductive wire member (W). The wire-guiding unit 110 has a space (i.e., a guide hollow 111 to be described below) formed therein through which the conductive wire member (W) passes. The wire-guiding unit 110 is formed to extend along a direction in which the conductive wire member (W) extends.

The wire-guiding unit 110 is coupled to the applying member accommodating unit 120. The conductive wire member (W) penetrating the wire-guiding unit 110 extends up to the applying member accommodating unit 120 and may be coupled to a stimulus applying unit 200 that is accommodated in the applying member accommodating unit 120 to conduct electricity.

The wire-guiding unit 110 may have any shape that is capable of supporting the conductive wire member (W) and guiding the same to the applying member accommodating unit 120. In the illustrated exemplary embodiment, the wire-guiding unit 110 has a square cross-section and extends in one direction.

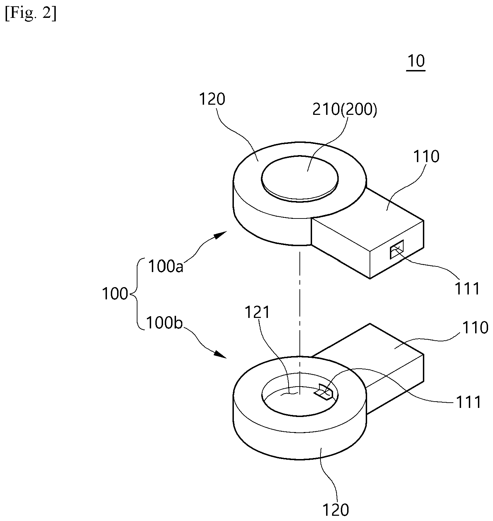

In this case, one pair of edges of the cross-section of the wire-guiding unit 110 may be formed to be longer than the other pair of edges. In the exemplary embodiment illustrated in FIG. 2, the cross-section of the wire-guiding unit 110 is formed with an extension length in the left and right directions that is longer than an extension length in the up and down directions.

Accordingly, it will be understood that among each surface of the wire-guiding unit 110, the upper or lower surface in the illustrated exemplary embodiment is formed to have a larger area, thereby making it easier to couple to the target portion (T).

A guide hollow 111 is formed to penetrate inside the wire-guiding unit 110.

The guide hollow 111 is a passage through which the conductive wire member (W) penetrates. The guide hollow 111 communicates with the exterior of the wire-guiding unit 110 and the accommodating space 121.

The guide hollow 111 is formed to extend in the same direction as the wire-guiding unit 110, that is, in the one direction. One end in the extension direction of the guide hollow 111, that is, the outer end, is formed to be open. In other words, the one end of the guide hollow 111 is formed to be open at one end facing outward among the ends in the extension direction of the wire-guiding unit 110. The one end of the guide hollow 111 communicates with the outside, and the conductive wire member (W) is inserted.

The other end, that is, the inner end, in the extension direction of the guide hollow 111 is also formed to be open. The other end of the guide hollow 111 is formed to be open at the other end that is coupled to the applying member accommodating unit 120 among the ends in the extension direction of the wire-guiding unit 110. The other end of the guide hollow 111 is in communication with the accommodating space 121, and is coupled to and electrically connected to the magnet member 210 that is accommodated in the accommodating space 121.

The guide hollow 111 may be of any shape through which the conductive wire member (W) can penetrate. In the illustrated exemplary embodiment, the guide hollow 111 has a square cross-section and is formed as a rectangular pillar-shaped space that is formed to extend along the extension direction of the wire-guiding unit 110.

The applying member accommodating unit 120 accommodates the stimulus applying unit 200, specifically the magnet member 210. Additionally, in an exemplary embodiment in which the stimulus applying apparatus 10 is provided in a wireless form, the applying member accommodating unit 120 may also accommodate a battery 230.

The applying member accommodating unit 120 is a part of the housing 100 that comes into contact with the target portion (T). The applying member accommodating unit 120 is formed to have as large a surface area as possible such that the state of contact with the target portion (T) can be stably maintained.

The applying member accommodating unit 120 is coupled to the wire-guiding unit 110. The conductive wire member (W) inserted into the guide hollow 111 of the wire-guiding unit 110 may extend up to the accommodating space 121 that is formed inside the applying member accommodating unit 120.

In this case, as described above, the first housing 100a and the second housing 100b may be arranged to face each other with the target portion (T) interposed therebetween. The first housing 100a and the second housing 100b may be in contact with different side surfaces of the target portion (T). In this case, the applying member accommodating unit 120 and the stimulus applying unit 200 accommodated therein may respectively contact the other side surfaces of the target portion (T).

Accordingly, it can be said that the applying member accommodating unit 120 forms a part of the stimulus applying apparatus 10 that contacts the target portion (T).

The applying member accommodating unit 120 accommodates the stimulus applying unit 200 and may have any shape that can be positioned adjacent to the target portion (T). In the illustrated exemplary embodiment, the applying member accommodating unit 120 has a circular cross-section and has a disk shape with a thickness in the vertical direction.

An accommodating space 121 is formed inside the applying member accommodating unit 120.

The accommodating space 121 accommodates the stimulus applying unit 200. The accommodating space 121 communicates with the guide hollow 111. The magnet member 210 of the stimulus applying unit 200 accommodated in the accommodating space 121 may be coupled to the control unit 220 through the conductive wire member (W) and conduct electricity.

The accommodating space 121 is formed to be recessed on one surface of the applying member accommodating unit 120 where the first housing 100a and the second housing 100b face each other. In the exemplary embodiment illustrated in FIG. 2, the accommodating space 121 is illustrated to be formed to be recessed in the upper surfaces of the first housing 100a and the second housing 100b.

However, this is for the convenience of understanding, and it will be understood that the accommodating spaces 121 are respectively formed on the lower surface of the first housing 100a and the upper surface of the second housing 100b, and are arranged to face each other.

Among the sides of the accommodating space 121, one side facing each other is open and communicates with the outside. The magnetic member 210 or the magnetic member 210 and the battery 230 may be accommodated in the accommodating space 121 through the one side.

The accommodating space 121 may have any shape that is capable of accommodating the magnet member 210 or the magnet member 210 and the battery 230. In the illustrated exemplary embodiment, the accommodating space 121 is formed as a disk-shaped space having a circular cross-section and a height in the vertical direction, similar to the applying member accommodating unit 120.

The stimulus applying unit 200 is configured to apply a stimulus to the target portion (T) to which the stimulus applying apparatus 10 is coupled. The stimulus applying unit 200 may be provided in any form that is capable of applying an appropriate stimulus to the target portion (T). In the illustrated exemplary embodiment, the stimulus applying unit 200 is configured to apply an electrical stimulus to the target portion (T).

The stimulus applying unit 200 may include a component for applying a stimulus to the target portion (T) and another component that is connected to the component and supplies power or a control signal.

In the illustrated exemplary embodiment, the stimulus applying unit 200 includes a magnet member 210, a control unit 220 and a battery 230.

The magnet member 210 is provided with the above configuration for applying a stimulus to the target portion (T). A stimulating electrode is provided inside the magnet member 210 such that an electrical stimulus can be applied to the target portion (T). The stimulating electrode may be configured to be electrically connected to the control unit 220 or the battery 230 to receive power and control signals.

The magnet member 210 is accommodated in the accommodating space 121 of the applying member accommodating unit 120. In this case, since one side of the accommodating space 121 is open, the magnet member 210 may be partially exposed to the outside of the applying member accommodating unit 120.

The magnet member 210 is provided in the first housing 100a and the second housing 100b, respectively. The magnet member 210 accommodated in the first housing 100a and the magnet member 210 accommodated in the second housing 100b are arranged to face each other with the target portion (T) interposed therebetween.

In this case, the magnet member 210 accommodated in the first housing 100a and the magnet member 210 accommodated in the second housing 100b may be configured to apply magnetic attractive force to each other. In other words, the magnetic members 210 accommodated in each housing 100a, 100b form magnetic attraction in a direction that attracts each other.

Accordingly, the magnet member 210 accommodated in the first housing 100a and the magnet member 210 accommodated in the second housing 100b that are disposed with the target portion (T) therebetween may be maintained in a state of being located adjacent to different surfaces of the target portion (T), respectively.

Therefore, as illustrated in FIG. 1, it will be understood that the stimulus applying apparatus 10 according to an exemplary embodiment of the present invention may be formed in the form of an earphone.

The magnetic member 210 forms magnetic attraction between each other, accommodates a stimulating electrode therein, and may be provided as an arbitrary form that is capable of delivering a stimulus generated by power and control signals transmitted through the stimulating electrode to the target portion (T). In an exemplary embodiment, the magnetic member 210 may be provided in the form of a neodymium magnet.

The magnetic member 210 is coupled to the conductive wire member (W) and conducts electricity. The stimulating electrode provided on the magnetic member 210 may be operated by receiving power and control signals from the external control unit 220.

The magnetic member 210 may be formed to correspond to the shape of the accommodating space 121. As described above, the accommodating space 121 is in the shape of a disk having a circular cross-section and a thickness in the vertical direction, and the magnet member 210 may also be formed in a disk shape having a circular cross-section and a thickness in the vertical direction.

In an exemplary embodiment in which the magnetic member 210 is formed in a disk shape, one surface of the magnetic member 210 that is exposed to the outside is located adjacent to the target portion (T) to be configured to apply a stimulus to the target portion (T). Additionally, in the above exemplary embodiment, the one surface of the magnetic member 210 may form magnetic attraction with another magnetic member 210 that is disposed to face each other with the target portion (T) therebetween.

The magnetic member 210 is coupled to the control unit 220 and the battery 230 and conducts electricity.

The control unit 220 transmits power and control signals for the stimulating electrode that is provided on the magnet member 210 to apply a stimulus to the target portion (T). The stimulating electrode may be operated by the delivered power and control signals to apply a stimulus to the target portion (T). In an exemplary embodiment, as described above, the magnetic member 210 may be configured to apply an electrical stimulus.

The control unit 220 conducts electricity with the magnetic member 210. In an exemplary embodiment in which the control unit 220 is provided on the outside of the housing 100, the control unit 220 may conduct electricity with the magnet member 210 through a conductive wire member (W). Accordingly, in the case of the above exemplary embodiment, it can be said that the stimulus applying apparatus 10 is provided in the form of a wired earphone.

The control unit 220 may be provided in any form that is capable of inputting, calculating and outputting information, and transmitting power. In an exemplary embodiment, the control unit 220 may include a microprocessor, CPU and the like as a configuration for the input, calculation and output of information. In addition, the control unit 220 may be configured by including a capacitor, so as to enable the storage or transmission of power.

In the above exemplary embodiment, the control unit 220 may include an input unit (not illustrated) for receiving information associated with control signals. The input unit (not illustrated) may be configured to allow a wearer or user to input information for controlling the operation of the stimulus applying apparatus 10 in any form. In an exemplary embodiment, the input unit (not illustrated) may be configured in the form of a button, dial or touch screen.

The battery 230 transmits power and control signals for the stimulating electrode that is provided on the magnet member 210 to apply a stimulus to the target portion (T). The stimulating electrode may be operated by the delivered power and control signals to apply a stimulus to the target portion (T).

A battery 230 is coupled to housing 100. The battery 230 may be accommodated in the accommodating space 121 formed in the applying member accommodating unit 120 together with the magnet member 210. **In** the above exemplary embodiment, the magnet member 210 may be configured to directly receive power and control signals from the battery 230. That is, in the above exemplary embodiment, the magnet member 210 does not need to conduct electricity with the external control unit 220 through the conductive wire member (W).

Accordingly, in the case of the above exemplary embodiment, it can be said that the stimulus applying apparatus 10 is provided in the form of a wireless earphone.

In this case, the battery 230 may be provided in any form that is capable of inputting, calculating and outputting information, and transmitting power, similar to the control unit 220. In an exemplary embodiment, the battery 230 may be configured by including a configuration for inputting, calculating and outputting information and a configuration for transmitting or storing power, similar to the control unit 220.

In the above exemplary embodiment, the size of the accommodating space 121 may be increased to accommodate more batteries 230. Alternatively, the size of the accommodating space 121 may be maintained, but the sizes of the magnet member 210 and the battery 230 may be miniaturized such that they are all accommodated in the accommodating space 121.

The stimulus applying apparatus 10 according to an exemplary embodiment of the present invention described above is provided with a pair of housings 100a, 100b that are arranged to face each other with the target portion (T) therebetween. A pair of housings 100a, 100b are respectively provided with a magnet member 210 including a stimulating electrode that applies a magnetic pole. The magnetic members 210 accommodated in each housing 100a, 100b are configured to form magnetic attraction toward each other.

Accordingly, the stimulus applying apparatus 10 may be maintained in a state where a pair of housings 100a, 100b are arranged to face each other with the target portion (T) therebetween, without a separate configuration for coupling. In addition, a stimulus may be applied to each side of the target portion (T) where the housings 100a, 100b are disposed to be adjacent to each other.

As a result, the stimulus applying apparatus 10 according to an exemplary embodiment of the present invention may achieve the intended effects by applying an appropriate stimulus to the target portion (T) without using an invasive method. In addition, the stimulus applying apparatus 10 may be easily attached to or removed from the target portion (T) by magnetic force.

In an exemplary embodiment, the stimulus applying apparatus 10 may be provided in a wired form. In the above exemplary embodiment, the magnet member 210 may be accommodated in the housing 100, and the control unit 220 may be disposed outside the housing 100. In this case, the magnet member 210 may be coupled to the control unit 220 and supplied with electricity through the conductive wire member (W). In the above exemplary embodiment, the stimulus applying apparatus 10 may be made more compact.

In another exemplary embodiment, the stimulus applying apparatus 10 may be provided in a wireless form. In the above exemplary embodiment, the magnetic member 210 is accommodated in the housing 100, and it may be coupled to and conduct electricity with the battery 230 that is also accommodated in the housing 100. In the above exemplary embodiment, the stimulus applying apparatus 10 may be worn easily.

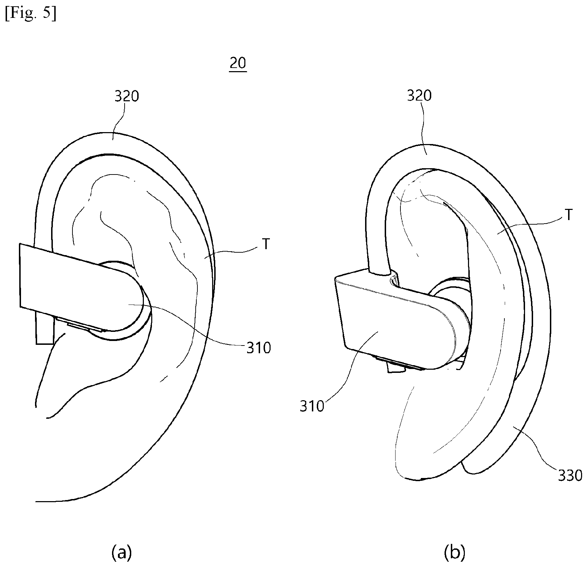

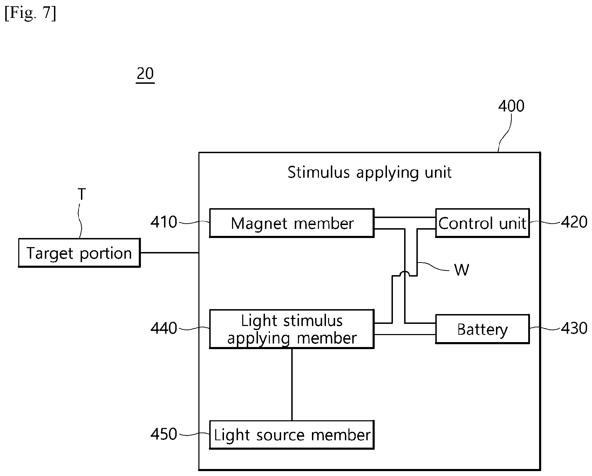

Referring to FIGS. 5 to 8, the stimulus applying apparatus 20 according to another exemplary embodiment of the present invention is illustrated. The stimulus applying apparatus 20 according to the illustrated exemplary embodiment may be detachably coupled to the target portion (T) and configured to apply a stimulus to the target portion (T).

Compared to the stimulus applying apparatus 10 according to the above-described embodiment, the difference in the stimulus applying apparatus 20 according to the present exemplary embodiment is that the housing 300 is formed as a single member and is coupled to the target portion (T).

In addition, the stimulus applying apparatus 20 according to the present exemplary embodiment is configured to apply not only an electrical stimulus applied to the target portion (T) by the stimulus applying apparatus 10 as described above, but also an optical stimulus.

Referring to FIG. 5, the target portion (T) to which the stimulus applying apparatus 20 according to the present exemplary embodiment is attached may be the ear area, particularly, the left ear area. Alternatively, the stimulus applying apparatus 20 according to the present exemplary embodiment may be coupled to any area where the holding member 320 can be mounted.

In the above exemplary embodiment, the stimulus applying apparatus 20 may be configured to apply stimuli to one side and the other side of the target portion (T), or to the outer side and inner side of the ear area in the illustrated exemplary embodiment. In other words, the stimulus applying apparatus 20 may be configured to apply stimuli to a part where the target portion (T) is exposed to the outer side (i.e., the one side) and a part that is hidden to the inner side (i.e., the other side).

In the illustrated exemplary embodiment, the stimulus applying apparatus 20 includes a housing 300 and a stimulus applying unit 400.

The housing 300 forms the external shape of the stimulus applying apparatus 20. The housing 300 is a part where the stimulus applying apparatus 20 is coupled to the target portion (T). The housing 300 may be formed to have a smaller size compared to the target portion (T).

The housing 300 may include a plurality of parts that are continuous with each other. The plurality of parts may be formed of different materials.

In this case, one part of the housing 300 may be made of a lightweight and highly rigid material. This is to prevent a situation where the stimulus applying apparatus 20 that is coupled to the target portion (T) is arbitrarily separated due to its self-weight.

In addition, other parts of the housing 300 may be formed of a softness material. As will be described below, the shape is changed to match the shape of the target portion (T) and is intended to be tightly coupled to the target portion (T).

The housing 300 may be formed of a non-conductive material. This is to prevent a situation where a stimulus to be delivered to the target portion (T) arbitrarily leaks into the housing 300.

In an exemplary embodiment, the one part of the housing 300 may be formed of a synthetic resin material such as insulating plastic. In addition, the other parts of the housing 300 may be formed of materials such as rubber and silicon.

The housing 300 is coupled to the conductive wire member (W). The stimulus applying unit 400 accommodated inside the housing 300 may be electrically connected to the outside through the conductive wire member (W).

The housing 300 is coupled to the stimulus applying unit 400. The stimulus applying unit 400 may be configured to apply a stimulus to the target portion (T) while being accommodated in the housing 300.

In the illustrated exemplary embodiment, the housing 300 includes a main housing 310, a holding member 320 and a sub-housing 330.

The main housing 310 forms a part of the outer shape of the housing 300. The main housing 310 is coupled to a part of the housing 300 and the target portion (T). In the illustrated exemplary embodiment, the main housing 310 covers the earhole and is coupled to the target portion (T).

The main housing 310 is exposed to the outer side of the target portion (T). In an exemplary embodiment in which the stimulus applying apparatus 20 is provided in the form of a wired earphone, the main housing 310 may be coupled to and conduct electricity with the control unit 420 that is provided externally through the conductive wire member (W).

The stimulus applying unit 400 is accommodated inside the main housing 310. one part of the target portion (T) located adjacent to the main housing 310 may receive a stimulus applied by the stimulus applying unit 400.

The main housing 310 is formed to have the largest volume among each configuration of the housing 300. At least a part of each configuration of the stimulus applying unit 400 may be accommodated inside the main housing 310.

In an exemplary embodiment in which the stimulus applying apparatus 20 is provided in the form of a wireless earphone, the main housing 310 may be provided with an indicator that displays the operating state of the stimulus applying apparatus 20.

In the illustrated exemplary embodiment, the main housing 310 is formed such that the extension length of the target portion (T) in the width direction is longer than the extension length of the target portion (T) in the height direction. The shape of the main housing 310 may be any shape that can be coupled to a part of the target portion (T) to apply a stimulus to the target portion (T).

In the illustrated exemplary embodiment, the main housing 310 includes an insertion protrusion unit 311.

The insertion protrusion unit 311 is a part of the main housing 310 that is inserted into an opening formed in the target portion (T). In an exemplary embodiment where the target portion (T) is the ear, the insertion protrusion unit 311 may be inserted into the ear hole.

The insertion protrusion unit 311 is formed on one side of the main housing 310 facing the target portion (T), which is the inner surface in the illustrated exemplary embodiment. When the stimulus applying apparatus 20 is coupled to the target portion (T), the insertion protrusion unit 311 is inserted into the opening and is not arbitrarily exposed to the outside.

A stimulus applying unit 400 is provided adjacent to the insertion protrusion unit 311. Specifically, a magnet member 410 and a light stimulus applying member 440 are provided in a part of the main housing 310 adjacent to the insertion protrusion unit 311. The magnet member 410 and the light stimulus applying member 440 are configured to apply an electrical stimulus and an optical stimulus to a part of the target portion (T) located adjacent to the insertion protrusion unit 311.

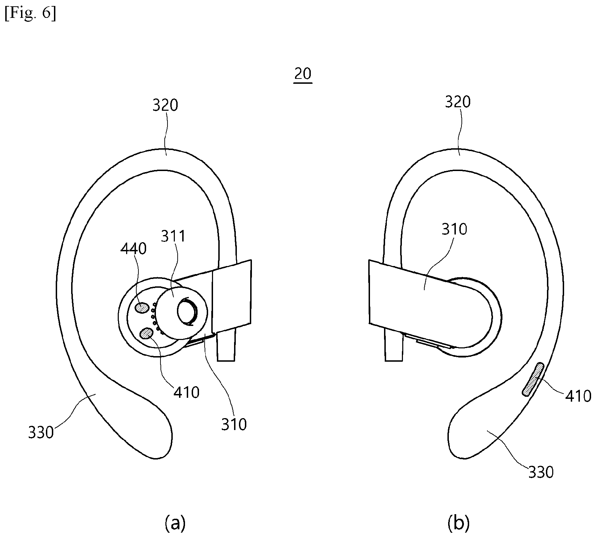

In the exemplary embodiment illustrated in FIG. 6, the magnet member 410 and the light stimulus applying member 440 are positioned between the insertion protrusion unit 311 and a continuous part of the holding member 320 and the sub-housing 330. In other words, the magnet member 410 and the light stimulus applying member 440 are located on a part of the inner side surface of the main housing 310.

The insertion protrusion unit 311 may have any shape that can be inserted into an opening formed in the target portion (T). In the illustrated exemplary embodiment, the insertion protrusion unit 311 has a circular cross-section and is provided in the form of an ear tip protruding toward the target portion (T).

The insertion protrusion unit 311 may be formed of a flexible material. After the shape is changed and inserted into an opening formed in the target portion (T), it is restored to its original shape and is maintained in the state of being inserted into the opening. In an exemplary embodiment, the insertion protrusion unit 311 may be formed of a silicon material.

The insertion protrusion unit 311 may be optionally provided. That is, in the illustrated exemplary embodiment, the stimulus applying apparatus 20 is provided with the insertion protrusion unit 311, but in other exemplary embodiments, the stimulus applying apparatus 20 may not be provided with the insertion protrusion unit 311. In any case, it is sufficient if the stimulus applying apparatus 20 can be stably coupled to the target portion (T).

The holding member 320 forms another part of the outer shape of the housing 300. The holding member 320 is a part on which the stimulus applying apparatus 20 is mounted on the target portion (T). The holding member 320 is continuous with the main housing 310 and is formed to be round and convex toward the outside. In other words, the holding member 320 is formed to be curved outward with its center on one side facing the main housing 310.

The holding member 320 is continuous with the main housing 310. The holding member 320 is electrically connected to the main housing 310 and may receive power and control signals applied by the battery 430 or the light source member 450 accommodated in the main housing 310.

The holding member 320 is continuous with the sub-housing 330. The holding member 320 is electrically connected to the sub-housing 330 such that the received power and control signals may be transmitted to the magnet member 410 accommodated in the sub-housing 330.

The holding member 320 extends between the main housing 310 and the sub housing 330. In the exemplary embodiment illustrated in FIG. 6, one end of the holding member 320 in the extension direction is coupled to the main housing 310 and conducts electricity. The other end of the holding member 320 in the extension direction is coupled to the sub-housing 330 and conducts electricity.

In the illustrated exemplary embodiment, the holding member 320 is formed to be rounded such that the upper side thereof is convex toward the outside. The other side of the round part of the holding member 320, which is the lower side in the illustrated exemplary embodiment, may be formed to be open. The holding member 320 may be mounted on the target portion (T) through the other side, that is, the lower side.

The holding member 320 may be formed of a flexible material. It changes shape according to the shape of the target portion (T) and is intended to be stably mounted on the target portion (T).

The outer shape of the holding member 320 may be made of a soft material. The holding member 320 is a part that directly contacts the target portion (T), and is intended to minimize the feeling of foreign matter.

In an exemplary embodiment, the holding member 320 may be formed of a silicon material.

The sub-housing 330 forms the remaining part of the outer shape of the housing 300. The sub-housing 330 is located adjacent to another part of the target portion (T). In an exemplary embodiment where the target portion (T) is the ear, the sub-housing 330 is located inside the ear.

A part of the stimulus applying unit 400 is accommodated in the sub-housing 330. The other part of the target portion (T) to which the sub-housing 330 is located adjacent may receive a stimulus applied by the stimulus applying unit 400 accommodated in the sub-housing 330.

The sub-housing 330 is coupled to the main housing 310 and the holding member 320 and conducts electricity. In the illustrated exemplary embodiment, the sub-housing 330 is positioned to be biased on one side, that is, the lower side where the main housing 310 is positioned to be biased. The sub-housing 330 is coupled to the other end in the extension direction of the holding member 320 and conducts electricity.

A space is formed inside the sub-housing 330. Some components of the stimulus applying unit 400, for example, the magnet member 410, may be accommodated in the space. The magnet member 410 accommodated inside the sub-housing 330 may apply a stimulus to the target portion (T) located adjacent thereto.

In the exemplary embodiment illustrated in FIG. 6, the magnet member 410 is arranged on the outer side surface of the sub-housing 330, that is, one side surface that is exposed to the outside when the stimulus applying apparatus 20 is not coupled to the target portion (T).

Power and control signals for operating the magnet member 410 accommodated in the sub-housing 330 may be applied by the configuration of the stimulus applying unit 400 accommodated in the main housing 310.

The stimulus applying unit 400 is configured to apply a stimulus to the target portion (T) to which the stimulus applicator 20 is coupled. The stimulus applying unit 400 may be provided in any form that is capable of applying an appropriate stimulus to the target portion (T). In the illustrated exemplary embodiment, the stimulus applying unit 400 is configured to apply an electrical stimulus or an optical stimulus to the target portion (T).

The stimulus applying unit 400 may include a component for applying a stimulus to the target portion (T) and another component that is electrically connected to the component and supplies power or control signals.

In the illustrated exemplary embodiment, the stimulus applying unit 400 includes a magnet member 410, a control unit 420, a battery 430, a light stimulus applying member 440 and a light source member 450.

The magnet member 410 is provided as a configuration for applying a stimulus to the target portion (T). In an exemplary embodiment, the magnet member 410 may be configured to apply an electrical stimulus to the target portion (T). A stimulating electrode is provided inside the magnet member 410 such that an electrical stimulus can be applied to the target portion (T). The stimulating electrode may be configured to be electrically connected to the control unit 420 or the battery 430 to receive power and control signals.

The magnetic member 410 is accommodated inside the housing 300. In this case, a plurality of magnet members 410 are provided, and the plurality of magnet members 410 may be accommodated in different parts of the housing 300, respectively. The plurality of magnet members 410 may be configured to respectively apply electrical stimuli to different parts of the target portion (T).

In the illustrated exemplary embodiment, two magnet members 410 may be provided and accommodated in the main housing 310 and the sub-housing 330, respectively. The magnetic member 410 accommodated in the main housing 310 may apply an electrical stimulus to a part of the target region T adjacent to the insertion protrusion unit 311. The magnet member 410 accommodated in the sub-housing 330 may apply an electrical stimulus to another part of the target portion (T) adjacent to the sub-housing 330.

In this case, as illustrated in FIG. 6, the plurality of magnet members 410 may be arranged to apply electrical stimuli in different directions. In the illustrated exemplary embodiment, the plurality of magnet members 410 are arranged to apply electrical stimuli to the outside and inside of the target portion (T).

That is, in the illustrated exemplary embodiment, the plurality of magnet members 410 may be arranged to surround the inside and outside of the target portion (T), respectively.

The magnetic member 410 forms magnetic attraction between each other, accommodates a stimulating electrode therein, and may be provided in an arbitrary form that is capable of delivering a stimulus generated by the power and control signals transmitted through the stimulating electrode to the target portion (T). In an exemplary embodiment, the magnetic member 410 may be provided in the form of a neodymium magnet.

The magnet member 410 is coupled to the conductive wire member (W) and conducts electricity. The stimulating electrode provided in the magnetic member 410 may be operated by receiving power and control signals from the external control unit 420.

The magnetic member 410 may be formed to have a smaller size than the main housing 310 or the sub-housing 330. In the above exemplary embodiment, the magnet member 410 may be accommodated inside the main housing 310 or the sub-housing 330 and may not be exposed to the outside.

The magnetic member 410 is coupled to the control unit 420 and the battery 430 and conducts electricity.

The control unit 420 transmits power and control signals for the stimulating electrode provided on the magnet member 410 to apply a stimulus to the target portion (T). The stimulating electrode may be operated by the transmitted power and control signals to apply a stimulus to the target portion (T). In an exemplary embodiment, as described above, the magnetic member 410 may be configured to apply an electrical stimulus.

In addition, the control unit 420 is coupled to the light source member 450 and conducts electricity. The control unit 420 transmits power and control signals to operate the light source member 450. The light source member 450 may generate light according to the received power and control signals and transmit the same to the light stimulus applying member 440.

The control unit 420 is electrically connected to the magnetic member 410. In an exemplary embodiment in which the control unit 420 is provided outside the housing 300, the control unit 420 may be electrically connected to the magnet member 210 through the conductive wire member (W). Accordingly, in the case of the above exemplary embodiment, it can be said that the stimulus applying apparatus 20 is provided in the form of a wired earphone.

The control unit 420 may be provided in any form that is capable of inputting, calculating and outputting information, and transmitting power. In an exemplary embodiment, the control unit 420 may include a microprocessor, CPU and the like for the input, calculation and output of information. In addition, the control unit 420 may be configured to include a capacitor to enable the storage or transmission of power.

In the above exemplary embodiment, the control unit 420 may include an input unit (not illustrated) for receiving information associated with control signals. The input unit (not illustrated) may be configured to allow a wearer or user to input information for controlling the operation of the stimulus applying apparatus 20 in any form. In an exemplary embodiment, the input unit (not illustrated) may be configured in the form of a button, dial or touch screen.

The battery 430 transmits power and control signals for the stimulating electrode provided on the magnet member 410 to apply a stimulus to the target portion (T). The stimulating electrode may be operated by the transmitted power and control signals to apply a stimulus to the target portion (T).

The battery 430 is accommodated in the housing 300. In an exemplary embodiment, the battery 430 may be accommodated inside the main housing 310 that has a relatively large volume. In the above exemplary embodiment, the magnet member 410 may be configured to directly receive power and control signals from the battery 430. That is, in the above exemplary embodiment, the magnet member 410 does not need to be energized with the external control unit 420 through the conductive wire member (W). The magnetic member 410 is coupled to the battery 430 and conducts electricity.

In addition, the battery 430 is coupled to the light source member 450 and conducts electricity. The battery 430 transmits power and control signals to operate the light source member 450. The light source member 450 may generate light according to the received power and control signals and transmit the same to the light stimulus applying member 440.

Accordingly, in the case of the above exemplary embodiment, it can be said that the stimulus applying apparatus 20 is provided in the form of a wireless earphone.

In this case, the battery 430 may be provided in any form that is capable of inputting, calculating and outputting information, and transmitting power, similar to the control unit 420. In an exemplary embodiment, the battery 430 may be configured to include a configuration for inputting, calculating and outputting information and a configuration for transmitting or storing power, similar to the control unit 420.

The light stimulus applying member 440 is provided in another configuration to apply a stimulus to the target portion (T). In an exemplary embodiment, the light stimulus applying member 440 may be configured to apply an optical stimulus to the target portion (T). The optical stimulus may be configured to be connected to the control unit 420 or the battery 430 to receive power and control signals.

The light stimulus applying member 440 may be coupled to the light source member 450, conduct electricity to receive light and be provided in any form that is capable of transmitting the same to the target portion (T). In an exemplary embodiment, the light stimulus applying member 440 may be provided in the form of an optical fiber.

The light stimulus applying member 440 is accommodated in the main housing 310. The light stimulus applying member 440 may be disposed adjacent to the insertion protrusion unit 311 to apply an optical stimulus to the target portion (T). The light stimulus applying member 440 is positioned adjacent to the magnet member 410 and the insertion protrusion unit 311 to which the magnet member 410 is disposed adjacently.

In FIG. 6, the light stimulus applying member 440 is illustrated as being provided only in the main housing 310. Alternatively, the light stimulus applying member 440 may be provided in the sub-housing 330 to apply an optical stimulus to the target portion (T) at a plurality of points.

In this case, the light stimulus applying member 440 provided in the sub-housing 330 may be arranged to apply an optical stimulus to the inner side of the target portion (T), similar to the magnet member 410. That is, the additionally provided light stimulus applying member 440 may be disposed adjacent to the magnet member 410 that is arranged in the sub-housing 330, as illustrated in (b) of FIG. 6.

The light stimulus applying member 440 is coupled to the control unit 420 or the battery 430 and conducts electricity. That is, in an exemplary embodiment in which the stimulus applying apparatus 20 is provided in the form of a wired earphone, the light stimulus applying member 440 may be coupled to the control unit 420 through the conductive wire member (W) and conduct electricity. In an exemplary embodiment in which the stimulus applying apparatus 20 is provided in the form of a wireless earphone, the light stimulus applying member 440 may be coupled to the housing 300, and specifically coupled to the battery 430 accommodated in the main housing 310, and conduct electricity.

The light source member 450 transmits light to the light stimulus applying member 440. The transmitted light may be provided to the target portion (T) in the form of an optical stimulus through the light stimulus applying member 440. The light source member 450 is coupled to the light stimulus applying member 440.

The light source member 450 is coupled to the light stimulus applying member 440. The light source member 450 is coupled to the control unit 420 or the battery 430 and conducts electricity. Power and control signals required for the light source member 450 to generate light may be transmitted from the control unit 420 or the battery 430.

In the illustrated exemplary embodiment, it is illustrated such that the main housing 310 is provided with the magnet member 410 and the light stimulus applying member 440, and the sub-housing 330 is provided with the magnet member 410.

Alternatively, the magnet member 410 and the light stimulus applying member 440 may be provided in at least any one of the main housing 310 and the sub-housing 330, respectively. In this case, the magnet member 410 and the light stimulus applying member 440 located in the main housing 310 or the sub-housing 330 may be arranged adjacent to each other.

In an exemplary embodiment in which the stimulus applying apparatus 20 is provided in the form of a wired earphone, the light source member 450 may be provided outside the housing 300. In the above exemplary embodiment, electricity connection between the light source member 450 and other members may be formed by the conductive wire member (W).

In an exemplary embodiment in which the stimulus applying apparatus 20 is provided in the form of a wireless earphone, the light source member 450 may be provided inside the housing 300, and specifically, the main housing 310. In the above exemplary embodiment, the light source member 450 and other members may be coupled directly to each other, may be electrically connected, or may be formed by the conductive wire member (W). In this case, it will be understood that the conductive wire member (W) is not exposed to the outside.

The stimulus applying apparatus 20 according to another exemplary embodiment of the present invention described above includes an insertion protrusion unit 311 that is inserted into the opening of the target portion (T) and a holding member 320 that is on the target portion (T). Accordingly, since the stimulus applying apparatus 20 is coupled to the target portion (T) at a plurality of positions, the coupled state of the stimulus applying apparatus 20 may be stably maintained.

In addition, the stimulus applying apparatus 20 may be configured to apply a stimulus to the target portion (T) from a plurality of points. In an exemplary embodiment, a plurality of magnet members 410 that apply an electrical stimulus to the target portion (T) may be provided in the insertion protrusion unit 311 and the sub-housing 330 of the main housing 310, respectively. The plurality of magnet members 410 may be configured to respectively apply electrical stimuli to different parts of the target portion (T).

In an exemplary embodiment, the stimulus applying apparatus 20 may be configured to apply different types of stimuli. In an exemplary embodiment, both of the magnet member 410 configured to apply an electrical stimulus to the target portion (T) and the light stimulus applying member 440 configured to apply an optical stimulus may be provided.

In the above exemplary embodiment, the magnet member 410 and the light stimulus applying member 440 may apply different types of stimuli to the target portion (T) at the same time or at different times.

As a result, the stimulus applying apparatus 20 according to an exemplary embodiment of the present invention may achieve the intended effects by applying appropriate stimuli to the target portion (T) without using an invasive method. In addition, the stimulus applying apparatus 20 may be easily attached to or removed from the target portion (T) by magnetic force.

In an exemplary embodiment, the stimulus applying apparatus 20 may be provided in a wired form. In the above exemplary embodiment, the magnet member 410 and the light stimulus applying member 440 may be accommodated in the housing 300, but the control unit 420 and the light source member 450 may be disposed outside the housing 300. In this case, the magnet member 410 and the light stimulus applying member 440 may be coupled to the control unit 420 or the light source member 450 and electrically connected to the conductive wire member (W). In the above exemplary embodiment, the stimulus applying apparatus 20 may be made more compact.

In another exemplary embodiment, the stimulus applying apparatus 20 may be provided in a wireless form. In the above exemplary embodiment, the magnet member 410 and the light stimulus applying member 440 are accommodated in the housing 300, and may be coupled to and conduct electricity with the battery 430 or the light source member 450 that is also accommodated in the housing 300. In the above exemplary embodiment, the stimulus applying apparatus 20 may be worn easily.

Hereinafter, the application aspects of the stimulus applying apparatuses 10, 20 according to the above-described exemplary embodiments of the present invention will be described in detail through examples. However, the following examples specifically illustrate the present invention, and the content of the present invention is not limited by the following examples.

### [Example 1]

### Construction of animal model to induce vascular dementia

In order to confirm the effects of the electroceutical of the present invention on recovering cognitive dysfunction, an animal model in which vascular dementia was induced was constructed.

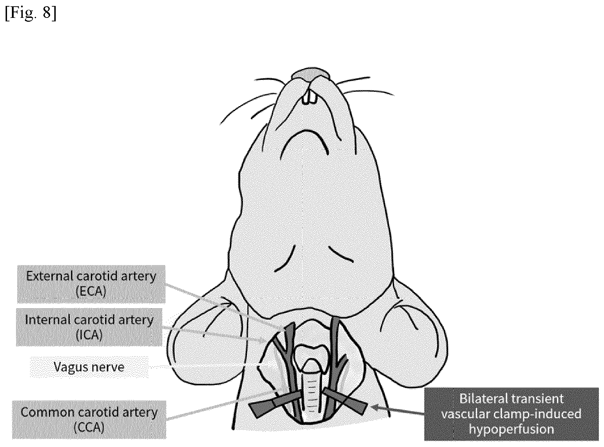

In order to induce vascular dementia, as illustrated in FIG. 8, 8-week-old C57BL/6 mice were anesthetized with isoflurane, and the anterior neck was incised. After the common carotid artery was exposed on both sides, it was temporarily clamped for 25 minutes to reduce cerebral blood flow by ligation (transient Bilateral Common Carotid Artery Occlusion, hereinafter, referred to as 'tBCCAO'). Afterwards, the surgical site was sutured (Soares et al., Behavioral Brain Research, 2013).

Experimental animals were obtained from Daehan Biolink, and all animals were raised in the Kyung Hee University laboratory animal room where a 12-hour light/dark cycle, constant temperature and constant humidity were maintained, and water and food were provided freely. The study was conducted in accordance with the guidelines of the Animal Experiment Ethics Committee of Kyung Hee University. All animal testing procedures followed the protocol of KHSASP-22-302 approved by the Animal Experiment Ethics Committee.

### [Example 2]

### Confirmation of heart rate reduction due to transcutaneous vagus nerve stimulus

Based on the fact that heart rate and related indicators (HRV, etc.) are presented as biomarkers to evaluate the effectiveness of bioregulation by vagus nerve stimulus (Saku et al., Physiological Reports, 2014; Gureal et al., Brain Stimulus, 2020), in order to confirm the operation of the parasympathetic nervous system and biological regulation according to the application of effective electrical stimuli, an electrocardiogram was taken during stimulus, and the heart rate per minute was tracked.

As a result, as illustrated in FIG. 8, it was confirmed that when the vagus nerve was stimulated, the heart rate decreased due to electrical stimuli.

### [Example 3]

Evaluation of memory impairment in vascular dementia induced by temporary common carotid artery ligation

In order to evaluate memory impairment induced in vascular dementia caused by common carotid artery ligation, the following two types of experiments were performed.

### <3-1> Object recognition test

In order to evaluate cognitive ability and memory impairment due to cognitive impairment, a novel object recognition test, which is a behavioral experiment that evaluates short-term memory for new objects, was performed.

The object recognition test was conducted in a cubic arena made of white acrylic measuring 40 cm × 40 cm × 40 cm. As objects, a blue cube object and a red square pyramid object made of plastic measuring 5 cm × 5 cm × 5 cm were used. The texture and volume of all objects were similar, and the experiment was conducted after confirming that there was no biased preference of the experimental animals. The object recognition test consisted of a habituation phase, a familiarization phase and an experiment phase. The activities of experimental animals within the arena were recorded on video. First of all, during the habituation phase, the animals were placed in an empty arena for about 10 minutes the day before the main test phase to allow them to adapt to the arena space, and it was evaluated whether there were any obstacles to the free movement function of the experimental animals. On the next day, during the familiarization phase, two identical objects were placed diagonally inside the arena, and the experimental animals were allowed to freely explore for 10 minutes. After one hour, in the test phase, the objects that had been explored in the familiarization period and the new objects were placed inside the arena, and the exploratory activity time of the experimental animals for each object was measured. Exploration activity was defined as when the experimental animal's nose was facing the object and the center of the experimental animal's body was about 2 cm away from the object.

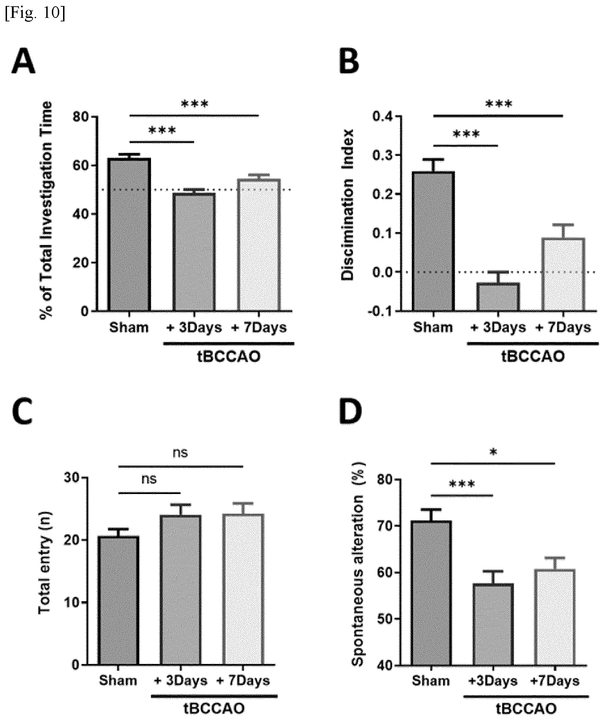

As a result of the experiments, as shown in FIGS. 10A and 10B, it was confirmed that the mice with vascular dementia induced by common carotid artery ligation had a significantly reduced time and frequency of showing interest in new objects compared to normal mice.

### <3-2> Y-maze

As an experiment to measure short-term memory and to evaluate the ability to act sequentially, the Y maze experiment, which is a behavioral experiment to evaluate working memory for space, was conducted.

The Y-maze device, which is a measuring device, consisted of a Y-shaped closed maze made of white acrylic plates [branches: 8 cm wide, 30 cm long, 14 cm high], and the angle at which the three branches folded was arranged at 120°. After determining each branch as A, B and C, the experimental animal was carefully placed on one branch, allowed to freely move through the Y-maze for 8 minutes, and each branch where the experimental animal entered was recorded through a camera installed on the ceiling. Spontaneous alteration (%) was evaluated by measuring the number and order of entries into each branch. An alternation was recognized as an actual alternation (i.e., the order of ABC, BCA and CAB) when it entered three different branches sequentially. If the entries were not performed consecutively, no point was acknowledged. Alteration behavior is defined as entering all three branches without overlapping, and spontaneous alteration was calculated by using the following mathematical formula. Therefore, the % alteration was calculated by using Mathematical Formula 1 below. % Alteration = [Total number of alterations] / [Total number of entries in branches - 2] * 100

As a result of the experiment, as shown in FIGS. 10C and 10D, the results of the Y-maze experiment confirmed that performance ability was significantly reduced due to memory impairment without causing motor function impairment on day 3 and day 7 after common carotid artery ligation surgery.

In other words, it was confirmed that performance ability was significantly reduced due to memory impairment on day 3 and day 7 after common carotid artery ligation surgery.

### [Example 4]

Recovery of cognitive function by transcutaneous vagal nerve stimulus (taVNS) in vascular dementia animals with memory impairment

In order to confirm whether repetitive and long-term transcutaneous vagus nerve stimuli restore cognitive function, behavioral evaluation was performed after applying stimuli under respiratory anesthesia.

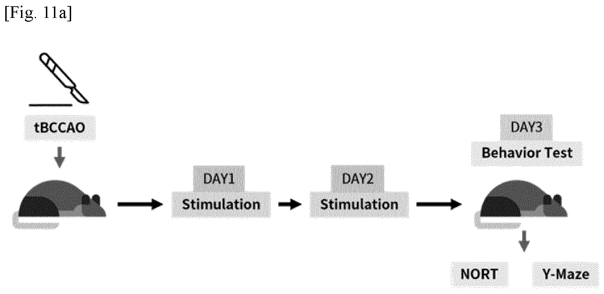

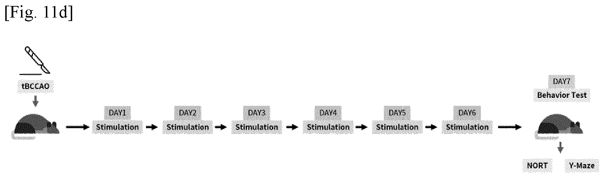

Specifically, vascular dementia was induced by transient Bilateral Common Carotid Artery Occlusion (tBCCAO), and then, stimuli were applied by the stimulus applying apparatuses 10, 20 to check whether cognitive function was recovered. The experimental performance plan therefor is shown in FIGS. 11a and 11d. Behavioral experiments on cognitive function were conducted by using the object recognition test and Y-maze test described above in Example 3. The control group was a group in which anesthesia was performed without stimulus (Anesthesia only).

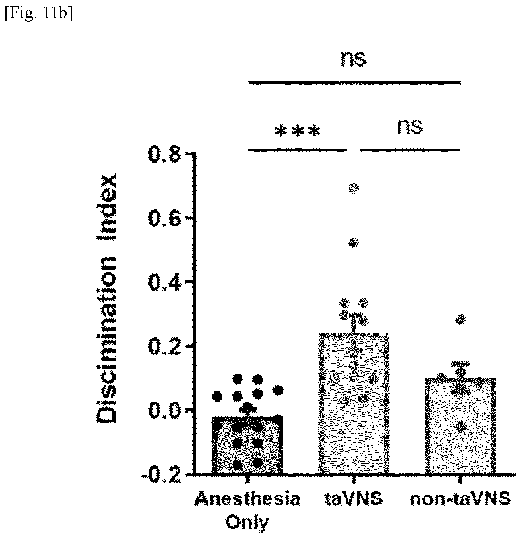

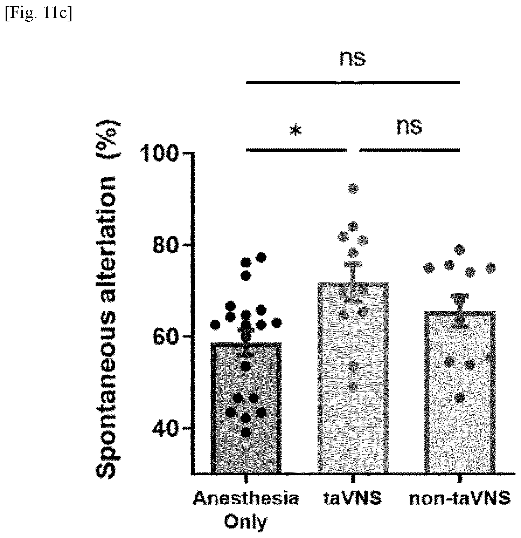

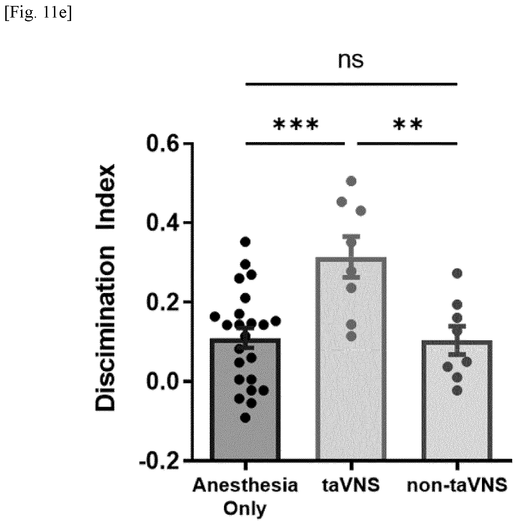

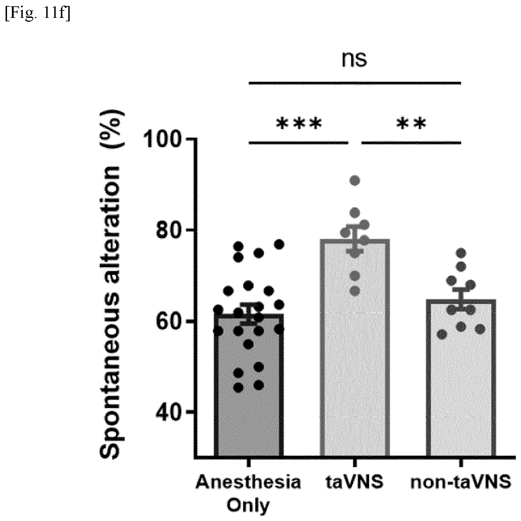

As a result, as a result of the behavioral evaluation on day 3 after surgery, it was confirmed that the performance ability of the object recognition test (FIG. 11b) and Y-maze (FIG. 11c) in the transcutaneous vagal nerve stimulus group (taVNS) was significantly increased compared to the group that received only anesthesia without stimulus. On the day 7 after surgery, there was a significant increase in the stimulus control group (non-taVNS), which confirmed the recovery of cognitive function (FIGS. 11e and 11f).

### [Example 5]

### Activation of cerebrospinal fluid circulation by transcutaneous vagus nerve stimulus

In order to explore the mechanism of cognitive function improvement in vascular dementia by transcutaneous vagus nerve stimulus, the effects on cerebrospinal fluid circulation were determined.

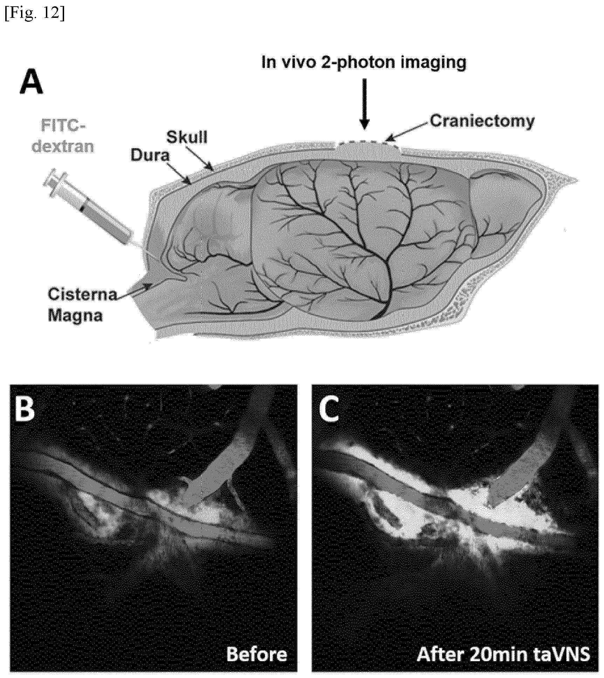

As shown in FIG. 12a, cerebrospinal fluid imaging was performed by anesthetizing a living subject and cutting the skull with a scalpel to a size for implanting a 2 mm × 2 mm transparent glass cover glass (Matsunami Glass, Osaka, Japan), and then, the cover glass was fixed on the cerebral cortex using a biobond (Vetbond, 3M) and dental cement. After securing an imaging window, the circulation of cerebrospinal fluid was observed by injecting a cerebrospinal fluid fluorescent tracer (FITC-dextran; Invitrogen) through the cisterna magna (Iliff et al., Science Translational Medicine, 2012).

As a result, it was confirmed that the intensity and area of fluorescence of the cerebrospinal fluid fluorescent tracer according to cerebrospinal fluid circulation increased after applying stimuli (FIG. 12c), compared to before percutaneous vagus nerve stimuli (FIG. 12b). As a result, it was confirmed that the transcutaneous vagus nerve stimulus activates cerebrospinal fluid circulation and improves cognitive function in vascular dementia.

| | | | |
|---|---|---|---|
| 10, 20: | Stimulus applying apparatus | 100: | Housing |
| 100a: | First housing | 100b: | Second housing |
| 110: | Wire-guiding unit | 111: | Guide hollow |
| 120: | Applying member accommodating unit | | |
| 121: | Accommodating space | | |
| 200: | Stimulus applying unit | 210: | Magnet member |
| 220: | Control unit | 230: | Battery |
| 300: | Housing | 310: | Main housing |
| 311: | Insertion protrusion unit | 320: | Holding member |
| 330: | Sub-housing | | |
| 400: | Stimulus applying unit | | |
| 410: | Magnet member | 420: | Control unit |
| 430: | Battery | | |
| 440: | Light stimulus applying member | | |
| 450: | Light source member | | |
| W: | Conductive wire member | | |
| T: | Target portion | | |

## Claims

1. A stimulus applying apparatus, comprising:
stimulus applying units which are configured to apply a stimulus to a target portion; and
a housing which is coupled to the stimulus applying unit,
wherein the housing comprises:
a first housing which is arranged on one side of the target portion and accommodates the stimulus applying unit; and
a second housing which is arranged on the other side of the target portion and accommodates the stimulus applying unit, and
wherein the stimulus applying unit accommodated in the first housing and the stimulus applying unit accommodated in the second housing are configured to apply magnetic attractive force to each other.

2. The stimulus applying apparatus of claim 1, wherein the stimulus applying unit comprises a magnetic member which is accommodated in the housing and comprises a stimulating electrode which is configured to apply the stimulus to the target portion, and
wherein the magnetic member is located to be biased on one side of the housing facing the target portion.

3. The stimulus applying apparatus of claim 2, wherein the housing comprises an applying member accommodating unit that accommodates the magnetic member and is located adjacent to the target portion, and
wherein the applying member accommodating unit comprises an accommodating space which is formed to be recessed on one surface facing the target portion (T) to accommodate the magnetic member.

4. The stimulus applying apparatus of claim 3, wherein the housing comprises a wire-guiding unit which is continuous with the applying member accommodating unit and is formed to extend in a direction opposite to the applying member accommodating unit, and
wherein the wire-guiding unit comprises guide hollows that are located therein and are formed to penetrate along an extension direction thereof such that one end in the extension direction communicates with the accommodating space, and the other end in the extension direction communicates with the outside.

5. The stimulus applying apparatus of claim 2, wherein the stimulus applying unit comprises a control unit which is electrically connected to the magnetic member and is configured to apply power and control signals to the magnetic member.

6. The stimulus applying apparatus of claim 5, wherein the control unit is located outside the housing, and comprises a conductive wire member which extends between the control unit and the magnet member and is configured to conduct electricity with the control unit and the magnet member, respectively.

7. The stimulus applying apparatus of claim 2, wherein the stimulus applying unit comprises a battery which is accommodated inside the housing to be adjacent to the magnetic member, and is configured to apply power and control signals to the magnetic member.

8. A stimulus applying apparatus, comprising:
stimulus applying units which are configured to apply a stimulus to a target portion; and
a housing which is coupled to the stimulus applying unit,
wherein the housing comprises:
a main housing which accommodates the stimulus applying unit, covers one side of the target portion, and is inserted into an opening that is formed in the target portion;
a sub-housing which accommodates the stimulus applying unit, and is located to be adjacent to the other side of the target portion; and
a holding member which extends between the main housing and the sub-housing, and is configured to be mounted on another side of the target portion.

9. The stimulus applying apparatus of claim 8, wherein the main housing comprises an insertion protrusion unit which is formed to protrude toward the target portion, and is inserted into the opening of the target portion, and
wherein the stimulus applying unit is arranged on the insertion protrusion unit, and is configured to apply the stimulus to a part that is adjacent to the opening of the target portion.

10. The stimulus applying apparatus of claim 8, wherein the stimulus applying unit comprises:
a magnetic member which comprises a stimulating electrode and is configured to apply an electrical stimulus to the target portion; and
a light stimulus applying member which is configured to apply an optical stimulus to the target portion.

11. The stimulus applying apparatus of claim 10, wherein the magnetic member is arranged in any one or more of the main housing and the sub-housing, and
wherein the light stimulus applying member is arranged to be adjacent to the magnet member in any one or more of the main housing and the sub-housing.

12. The stimulus applying apparatus of claim 10, wherein the magnetic member is arranged in any one or more of the main housing and the sub-housing, and
wherein the light stimulus applying member is arranged to be adjacent to the magnet member in any one or more of the main housing and the sub-housing.

13. The stimulus applying apparatus of claim 9, wherein the stimulus applying unit comprises:
a magnetic member which comprises a stimulating electrode and is configured to apply an electrical stimulus to the target portion; and
a control unit which is electrically connected to the magnetic member and is configured to apply power and control signals to the magnetic member,
wherein the control unit is arranged outside the housing and is configured to conduct electricity to the magnet member by a conductive wire member.

14. The stimulus applying apparatus of claim 9, wherein the stimulus applying unit comprises:
a magnet member which is accommodated in the housing, comprises a stimulating electrode and is configured to apply an electrical stimulus to the target portion; and
a battery which is accommodated in the housing to be adjacent to the magnetic member, is electrically connected to the magnetic member and is configured to apply power and control signals to the magnetic member.
